(19) **Europäisches Patentamt**
**European Patent Office**
**Office européen des brevets**

(11) Veröffentlichungsnummer : **0 445 710 B1**

(12) # EUROPÄISCHE PATENTSCHRIFT

(45) Veröffentlichungstag der Patentschrift :
**02.02.94 Patentblatt 94/05**

(51) Int. Cl.$^5$ : **A61K 39/39**

(21) Anmeldenummer : **91103258.9**

(22) Anmeldetag : **05.03.91**

(54) **Verwendung von Zink-Calciumhydroxid, Lecithin und PAO zur Adjuvierung von Antigenlösungen und auf diese Weise adjuvierte Antigenlösungen.**

(30) Priorität : **08.03.90 DE 4007315**

(43) Veröffentlichungstag der Anmeldung :
**11.09.91 Patentblatt 91/37**

(45) Bekanntmachung des Hinweises auf die
Patenterteilung :
**02.02.94 Patentblatt 94/05**

(84) Benannte Vertragsstaaten :
**AT BE CH DE DK ES FR GB GR IT LI LU NL SE**

(56) Entgegenhaltungen :
**EP-A- 0 343 548**
**EP-A- 0 363 835**
**DE-A- 3 712 768**
**US-A- 4 552 756**

(73) Patentinhaber : **BEHRINGWERKE**
**Aktiengesellschaft**
**Postfach 1140**
**D-35001 Marburg (DE)**

(72) Erfinder : **Bernhardt, Dieter, Dr.**
**Goldbergstrasse 18a**
**W-3553 Cölbe (DE)**
Erfinder : **Hilfenhaus, Joachim, Dr.**
**Auf'm Gebrande 24**
**W-3550 Marburg (DE)**

## Beschreibung

Die Erfindung betrifft die Verwendung von Zink-Calciumhydroxid, Lecithin und PAO zur Adjuvierung von Antigenlösungen sowie auf diese Weise adjuvierte Antigenlösungen.

Viele Antigene sind so wenig immunogen, daß sie nach einmaliger Injektion in ein Tier keine meßbare oder eine nur geringe Immunantwort auslösen. Um die Immunantwort des Organismus auf einen Antigenreiz zu verstärken, werden deshalb dem Antigen Adjuvantien zugesetzt. Die meisten inaktivierten Virus- und Bakterienvaccinen enthalten aus diesem Grund Adjuvantien. In diesen Virus- und Bakterienimpfstoffen benutzt man überwiegend $Al(OH)_3$ und $AlPO_4$, einzeln oder in Kombination, Pflanzenöle oder aus Erdölfraktionen gewonnene Mineralöle, sogenannte medizinisch pharmazeutische Weißöle nicht exakt definierter Zusammensetzung. Freund's komplettes und inkomplettes Adjuvans enthaltend Mineralöl [R]Bayol F mit und ohne Extrakt von Mycobakterien, wird vorwiegend in experimentellen Vaccinen eingesetzt.

Diese Adjuvantien können aber neben Lokalreaktionen auch systemische Nebenwirkungen entfalten.

Neben der lokalen und allgemeinen Verträglichkeit von Adjuvantien sind von Interesse:

1. Ihre immunologischen Wirkmechanismen, also die Wirkung, die sie induzieren;

2. Ihre Pharmakokinetik (Bioabbaubarkeit).

Zu 1: So ist allgemein bekannt, daß die mineralischen Adjuvantien ($Al(OH)_3$, $AlPO_4$) vorwiegend nur eine humorale Immunantwort induzieren, während die zelluläre Immunität, die bei vielen Virusinfektionen eine dominierende Rolle spielt, nur geringfügig oder gar nicht stimuliert wird.

Anders verhalten sich die Mineralöle und besonders Freund's Adjuvans, die bekanntermaßen sowohl die zelluläre als auch die humorale Immunantwort stimulieren.

Zu 2: Die bisher gebräuchlichen Adjuvantien verbleiben größtenteils an der Injektionsstelle oder werden abtransportiert und in anderen Organen des Organismus angereichert, wo sie ihre immunologische und gegebenenfalls toxische Wirkung entfalten, d. h. ein Abbau und eine Ausscheidung findet nicht oder nur sehr verzögert statt.

Anders verhalten sich die Komponenten des erfindungsgemäßen Adjuvans, nämlich Zinkhydroxid-Calciumhydroxid-Gel und Lecithin. Diese unterliegen im Organismus dem Stoffwechsel. Sie sind daher weniger toxisch.

In EP-A-0 108 316 (deutsche Offenlegungsschrift 32 41 113) wurde vorgeschlagen, neben anderen Stoffen Verbindungen des Zinks, und zwar Zinksalze als Zuschlagstoffe zu Vaccinen zu verwenden.

In EP-A-0 343 548 sind Polyalphaolefine (PAO) und in EP-A-0 363 835 Zink-Hydroxidgel und Lecithin als Adjuvantien beschrieben.

Überraschenderweise wurde aber nun gefunden, daß Zinkhydroxid-Calciumhydroxid-Gele, Lecithin und PAO sich zu einem Adjuvans kombinieren lassen, das folgende Eigenschaften aufweist:

1. Das kombinierte Adjuvans hat eine erheblich bessere immunstimulierende Wirkung als jede der 4 adjuvierend wirkenden Einzelkomponenten.

2. Das kombinierte Adjuvans hat überraschenderweise eine bessere allgemein und lokale Verträglichkeit als Zink-Hydroxidgel und Lecithin oder PAO.

Verfahrensbeschreibung der Herstellung von Zinkhydroxid/ Calciumhydroxid-Gel und Lecithin 99-Suspension nach an sich bekannten Verfahren:

Zinkhydroxid/Calciumhydroxid-Gel:

1. Ausgehend von $ZnCl_2$ und $CaCl_2$ x 2 $H_2O$, Herstellung einer 0,1 M-Lösung in Aqua dest. (jeweils 0,1 mol/l);

2. Sterilfiltration der ZnCa-Salzlösung (0,2 μ Membranfilter);

3. Unter sterilen Bedingungen und unter Rühren erfolgt die Zugabe einer Base, vorzugsweise von 10 N NaOH oder 10 N KOH bis ein pH von 6,8 - 7,8 erreicht ist;

4. Das ausgefallene Zinkhydroxid-Calciumhydroxid-Gel kann vorzugsweise durch [R]Ultraturax-Behandlung homogenisiert werden.

Die hier als Ausgang benutzten ZnCa-Salze sind nur beispielhaft. Ebenso ist es möglich, ein Zink-hydroxid-Calciumhydroxid-Gel unter pH-Kontrolle direkt in einer Antigensuspension herzustellen. Arbeitet man nicht mit sterilen ZnCa-Salzlösungen, sondern unter unsterilen Bedingungen, kann das Gel 20 min bei 120° C autoklaviert werden.

Herstellung einer 20%igen Lecithin 99-Suspension:

1. 20 g Lecithin werden in 100 ml PBS (phosphatgepufferte Salzlösung nach Dulbeco), pH 7,2 suspendiert;

2. Autoklavieren der Suspension 20 min bei 120° C;
3. Nach Abkühlen wird die Suspension homogenisiert;
4. pH-Einstellung der Suspension mit 10 N NaOH auf 7,0 - 7,8.
Beispielsweise Herstellung einer erfindungsgemäßen Adjuvanskombination (BW 89):

| | |
|---|---|
| PAO | 26.67 % |
| werden mit $^R$Tween 81 und $^R$Tween 80 | 6.00 % |
| (beides Polyoxyethylensorbitan-monoester) | 2.00 % |
| mittels eines Homogenisators gut gemischt. | |
| Unter $^R$Ultraturaxmischung erfolgt die | |
| Zugabe von Zink/Calciumhydroxid-Gel, 0.1 M | 50.00 % |
| und Lecithin 99, 20 %ige Suspension | 15.33 % |
| | ========= |
| | 100.00 % |

Die so erhaltene Adjuvanskombination kann in jedem beliebigem %-Satz einem Antigen zugesetzt werden.
Das Lecithin wird vorzugsweise als 20%ige Suspension zugegeben. Von einer solchen Suspension können 1-10 %, vorzugsweise 5 % zugesetzt werden.
Das Polyalphaolefin wird zu einer Konzentration von 1-40 %, vorzugsweise 10 % zugegeben.
Eine andere Möglichkeit der Adjuvanskombinationsherstellung ist:
1. Die Zugabe von Zink/Calciumhydroxid-Gel und Lecithin 99-Suspension zum Antigen und
2. Herstellung der W/O Emulsion (Wasser in Öl-Emulsion) mit PAO, Tween 81 und 80 mit der unter 1 beschriebenen Antigen-Adjuvansmischung.
Die Herstellung einer erfindungsgemäßen Adjuvanskombination kann aber auch auf diese Weise erfolgen, wobei alle Arbeitsschritte unter sterilen Bedingungen durchgeführt werden:

| | |
|---|---|
| 1. PAO | 26.67 % |
| $^R$Tween 80 | 6.00 % |
| $^R$Tween 81 | 2.00 % |
| werden mittels Ultraturax gut gemischt. | |
| 2. Unter Zugabe einer sterilen Lösung von | |
| $ZnCl_2/CaCl_2$, 0.1 M, | 50.00 % |
| und Mischen mittels $^R$Ultraturax erfolgt | |
| die Herstellung einer Wasser-in-öl(W/O)- | |
| Emulsion. | |

3. Unter pH-Kontrolle (6,5 - 7,5) erfolgt durch Zugabe von NaOH, 10 N, unter Mischen mit dem Ultraturax, die Ausfällung von Zink/Calciumhydroxid-Gel in der W/O-Emulsion.

4. Danach erfolgt unter Mischen die Zugabe von Lecithin 99, 20%ig             15.33 %

        **Gesamt**                      **100.00 %**

Eine andere Möglichkeit der Adjuvanskombinationsherstellung ist:
1. Die Zugabe von Zink/Calciumhydroxid-Gel und Lecithin 99 - Suspension zum Antigen und adsorbierenlassen des Antigens und
2. Herstellung der W/O-Emulsion mit PAO, [R]Tween 81 und 80 mit der unter 1 erwähnten Antigen-Adjuvansmischung.
Die folgenden Beispiele erläutern die Vorteile der erfindungsgemäßen Adjuvanskombination:

**Beispiel 1:**

Aujeszky disease virus (AV) wurde in primären Schweinenierenzellkulturen vermehrt. Nachdem die Kulturen 100%ig virusspezifisch zerstört worden waren, das heißt, es wurde gewartet, bis die Zellen vollständig zerstört waren (CPE), wurde das Virus geerntet und durch Zentrifugation und Filtration gereinigt. Hiernach erfolgte die AV-Inaktivierung mit Äthylenimin. Nach Sterilitäts- und Safetyprüfung wurden aus diesem inaktivierten AV-Antigen vier Vaccinen hergestellt.
Die Zusammensetzung der Vaccine ist nachfolgend in Tabelle 1 aufgeführt (Angaben in ml):

## Vaccine

| Adjuvans/Antigen | A | B | C | D |
|---|---|---|---|---|
| Al(OH)$_3$, 2%ig | 20 | | | |
| Zn(OH)$_2$, 0.1M | | 17 | | |
| Lecithin 99, 20%ig | | 3 | | |
| PAO | | | 20 | |
| BW 89 Adjuvanskombination | | | | 20 |
| AV-Antigen | 80 | 80 | 80 | 80 |
| Gesamt | 100 | 100 | 100 | 100 |

Zur Feststellung der lokalen Verträglichkeit wurden mit jeder Vaccine 2 Meerschweinchen, 450 g schwer, mit je 0,1 ml intraplantar geimpft.
Die Planten der Meerschweinchen wurden nach Impfung täglich 4 Wochen lang zur Beurteilung der lokalen

4

Verträglichkeit auf Rötung, Schwellung und andere sichtbare pathologisch, anatomische Veränderungen untersucht, und somit ein Punktwert der Unverträglichkeit ermittelt. Je schwerer die sichtbaren pathologisch, anatomischen Veränderungen sind, je höher ist der Punktwert, desto größer ist die Unverträglichkeit.

Die ermittelten Unverträglichkeitspunkte sind nachfolgend tabellarisch aufgeführt:

## Vaccine

| | A | B | C | D |
|---|---|---|---|---|
| Unverträglichkeitspunkte Tier 1 | 245 | 122,5 | 42,5 | 2,5 |
| Unverträglichkeitspunkte Tier 2 | 257 | 187,5 | 82,5 | 2,5 |
| Unverträglichkeitspunkte gesamt | 502 | 310,0 | 125,0 | 5,0 |

Wie aus den Resultaten der Tabelle hervorgeht, ist die BW 89 Adjuvanskombination (Vaccine D) allen anderen Adjuvantien hinsichtlich lokaler Verträglichkeit weit überlegen.

Zur Beurteilung der Wirksamkeit der Vaccinen wurden den Meerschweinchen 4 Wochen p.v. Blut entnommen, und im Serum die neutralisierenden Antikörper gegen das AV bestimmt.

Die ermittelten Neutralisationstiter sind nachfolgend tabellarisch aufgeführt.

## Vaccine

| Tier | A | B | C | D |
|---|---|---|---|---|
| Tier 1 | < 2 | 1 : 6 | 1 : 10 | 1 : 21 |
| Tier 2 | < 2 | 1 : 8 | 1 : 12 | 1 : 24 |

Wie die Resultate in dieser Tabelle zeigen, ist die Wirksamkeit der Adjuvanskombination (Vaccine D) besser als die der anderen Adjuvantien.

**Bestimmung der allgemeinen Verträglichkeit:**

Jeweils 10, 30g schwere NMRI Mäuse wurden mit den Vaccinen, die wie oben beschrieben hergestellt worden waren, mit je 0,3 ml s.c. vacciniert.

Zur Bestimmung der allgemeinen Verträglichkeit wurde bei den Tieren bis 8 Tagen p. v. täglich eine Gewichtskontrolle durchgeführt.

Die Gewichtszunahme bei den Tieren betrug mit

| | |
|---|---|
| Vaccine A | 11 g |
| Vaccine B | 6 g |
| Vaccine C | 8 g |
| Vaccine D | 11 g |
| unbehandelte Kontrollen | 10 g. |

Die Gewichtszunahme zeigt klar, daß die Adjuvanskombination BW 89 eine gute allgemeine Verträglichkeit aufweist.

**Beispiel 2:**

$PI_3$-(Parainfluenza 3)-Virus wurde in DBK-Zellen vermehrt. Nachdem der Zellrasen der Kulturen 80 - 100 %ig virusspezifisch zerstört worden war, wurde geerntet und mittels Zentrifugation gereinigt. Hiernach erfolgte die Inaktivierung des Virusantigens mit Ethylenimin. Nach Sterilitäts- und Safetyprüfung wurden aus dem inaktivierten Antigen 2 Vaccinen hergestellt;

Vaccine A enthielt (die %-Angaben zu den Vaccine-Zusammensetzungen sind als v:v zu verstehen):

| | |
|---|---|
| PI$_3$V - Antigen | 80.0 % |
| Al(OH)$_3$, 2%ig | 19.5 % |
| Saponin, Merck, 10%ig | 0.5 % |

Vaccine B enthielt:

| | |
|---|---|
| PI$_3$V - Antigen | 80.0 % |
| BW 89 | 20.0 % |

Mit beiden Vaccinen wurden je 3 Schafe mit 2.0 ml s.c. vacciniert und 4 Wochen p. v. mit der gleichen Dosis revacciniert. Zur Prüfung der lokalen Verträglichkeit der Vaccine wurde die Injektionsstelle auf Schwellungen, Rötungen, Konsistenz, Schmerzhaftigkeit untersucht.

Bei der Vaccine A war 8 Wochen p. v. immer noch ein Impfknoten visibel, dessen Rückbildung auf Grund seiner festen Konsistenz noch mehrere Wochen benötigt. Wesentlich besser lokal verträglich war die Vaccine B. Zwischen 14 und 21 Tagen p. v. war an der Infektionsstelle kein Impfknoten mehr sicht- und palpierbar. Keines der Tiere, die mit Vaccine A oder B geimpft worden waren, zeigte Störungen des Allgemeinbefindens.

**Beispiel 3**

Zur Prüfung auf Verträglichkeit wurden 2 Blindvaccinen (Blindvaccinen enthalten kein Antigen; anstelle des Antigens wird Eagles 59 Medium zugefügt. Der Adjuvansgehalt ist aber gleich, wie in der Vaccine vorgesehen) nachfolgender Zusammensetzung hergestellt;

Vaccine A enthielt:

```
EB (physiologische Salzlösung
      + Vitamine + Aminosäuren)            90.0 %
   Al(OH)₃, 2%ig                           10.0 %
```

Vaccine B enthielt:

| | |
|---|---|
| EB | 90.0 % |
| BW 89 | 10.0 % |

Mit Vaccine A wurden 3, mit Vaccine B wurden 6 Pferde s.c. mit 2.0 ml geimpft.

Die Tiere wurden 8 Tage lang täglich auf Lokalreaktionen untersucht. Die Resultate sind nachstehend tabellarisch zusammengefaßt.

**Lokalreaktionen bei Pferden nach Vaccination mit Al(OH)₃ oder BW 89 enthaltenden Blindvaccinen**

| Tier Nr. | Vaccine | Lokalreaktionen in Tagen p. v. | | | | | | | |
|---|---|---|---|---|---|---|---|---|---|
| | | 1 | 2 | 3 | 4 | 5 | 6 | 7 | 8 |
| 1 | | C | D | D | C | C | C | C | C |
| 2 | A | C | C | D | D | D | D | C | C |
| 3 | | C | D | D | D | C | C | C | C |
| 4 | | O | O | O | O | O | O | O | O |
| 5 | | O | O | O | O | O | O | O | O |
| 6 | B | A | A | B | O | O | O | O | O |
| 7 | | A | A | O | O | O | O | O | O |
| 8 | | C | O | O | O | O | O | O | O |
| 9 | | C | O | O | O | O | O | O | O |

Schwellungsgröße: O = nicht sichtbar, nicht palpierbar

A = erbsengroß

B = haselnußgroß

C = walnußgroß

D = hühnereigroß

Wie aus dieser Tabelle hervorgeht, ist BW 89 lokal wesentlich verträglicher als Al(OH)₃. Nennenswerte Allgemeinreaktionen traten bei keinem Tier auf.

**Patentansprüche**

**Patentansprüche für folgende Vertragsstaaten : AT, BE, CH, DE, DK, FR, GB, IT, LI, LU, NL, SE**

1. Verwendung von Zinkhydroxid-Calciumhydroxid-Gel, einem Lecithin und einem Polyalphaolefin in einem Verfahren zur Adjuvierung einer Antigenlösung.

2. Antigenlösung adjuviert nach Anspruch 1, dadurch gekennzeichnet, daß der Antigenlösung Zinkhydroxid-Calciumhydroxid-Gel, 1 - 10 %, vorzugsweise 5 %, einer 20 %igen Lecithinsuspension und 1 - 40 %, vorzugsweise 10 %, eines Polyalphaolefins zugegeben worden sind.

3. Verfahren zur Herstellung einer adjuvierten Antigenlösung, dadurch gekennzeichnet, daß einer Antigenlösung Zinkhydroxid-Calciumhydroxid-Gel, ein Lecithin und ein Polyalphaolefin zugegeben werden.

**Patentansprüch für folgende Vertragsstaaten : ES, GR**

1. Verfahren zur Herstellung einer adjuvierten Antigenlösung, dadurch gekennzeichnet, daß einer Antigenlösung Zinkhydroxid-Calciumhydroxid-Gel, ein Lecithin und ein Polyalphaolefin zugegeben werden.

7

## Claims

### Claims for the following Contracting States : AT, BE, CH, DE, DK, FR, GB, IT, LI, LU, NL, SE

1. The use of zinc hydroxide/calcium hydroxide gel, a lecithin and a polyalphaolefin in a process for treating an antigen solution with adjuvant.

2. An antigen solution treated with adjuvant as claimed in claim 1, to which antigen solution zinc hydroxide/calcium hydroxide gel, 1-10 %, preferably 5 %, of a 20 % strength lecithin suspension and 1-40 %, preferably 10 %, of a polyalphaolefin have been added.

3. A process for preparing an antigen solution treated with adjuvant, which comprises adding zinc hydroxide/calcium hydroxide gel, a lecithin and a polyalphaolefin to an antigen solution.

### Claim for the following Contracting States : ES, GR

1. A process for preparing an antigen solution treated with adjuvant, which comprises adding zinc hydroxide/calcium hydroxide gel, a lecithin and a polyalphaolefin to an antigen solution.

## Revendications

### Revendications pour les Etats contractants suivants : AT, BE, CH, DE, DK, FR, GB, IT, LI, LU, NL, SE

1. Emploi d'un gel d'hydroxyde de zinc et d'hydroxyde de calcium, d'une lécithine et d'une polyalphaoléfine dans un procédé d'adjuvation d'une solution d'antigène.

2. Solution d'antigène adjuvée selon la revendication 1, caractérisée en ce que l'on ajoute à la solution d'antigène un gel d'hydroxyde de zinc et d'hydroxyde de calcium, 1 à 10 %, de préférence 5 % d'une suspension de lécithine à 20 % et 1 à 40 %, de préférence 10 %, d'une polyalphaoléfine.

3. Procédé de préparation d'une solution d'antigène adjuvée, caractérisé en ce que l'on ajoute à une solution d'antigène un sel d'hydroxyde de zinc et d'hydroxyde de calcium, une lécithine et une polyalphaoléfine.

### Revendication pour les Etats contractants suivants : ES, GR

1. Procédé de préparation d'une solution d'antigène adjuvée, caractérisé en ce que l'on ajoute à une solution d'antigène un sel d'hydroxyde de zinc et d'hydroxyde de calcium, une lécithine et une polyalphaoléfine.